**Europäisches Patentamt**

**European Patent Office**

**Office européen des brevets**

(11) Veröffentlichungsnummer: **0 081 655**
**B1**

(12) ## EUROPÄISCHE PATENTSCHRIFT

(45) Veröffentlichungstag der Patentschrift:
**25.09.85**

(21) Anmeldenummer: **82109546.0**

(22) Anmeldetag: **15.10.82**

(51) Int. Cl.⁴: **A 61 M 1/00**, A 61 M 1/02,
A 61 M 5/00

(54) **Entlüftungseinrichtung für ein medizinisches Flüssigkeitssystem.**

(30) Priorität: **01.12.81 DE 3147499**

(43) Veröffentlichungstag der Anmeldung:
**22.06.83 Patentblatt 83/25**

(45) Bekanntmachung des Hinweises auf die Patenterteilung:
**25.09.85 Patentblatt 85/39**

(84) Benannte Vertragsstaaten:
**AT BE CH DE FR GB IT LI LU NL SE**

(56) Entgegenhaltungen:
**DE - A - 3 011 681**
**GB - A - 1 510 072**
**GB - A - 2 042 918**
**GB - A - 2 061 125**
**US - A - 3 982 538**

(73) Patentinhaber: **INTERMEDICAT GMBH,**
**Gerliswilstrasse 74, CH-6020 Emmenbrücke (CH)**

(72) Erfinder: **Herlitze, Gerhard, Bindsdorfer Strasse 4,**
**D-3507 Baunatal (DE)**

(74) Vertreter: **von Kreisler, Alek, Dipl.-Chem. et al,**
**Deichmannhaus am Hauptbahnhof, D-5000 Köln 1 (DE)**

## Beschreibung

Die Erfindung betrifft eine Entlüftungseinrichtung für ein medizinisches Flüssigkeitssystem, mit einer das Flüssigkeitssystem abschliessenden, luftdurchlässigen hydrophoben Schicht und mit einer auf der der Flüssigkeit zugewandten Seite der hydrophoben Schicht angeordneten Schicht aus hydrophilem Material, die bei Benetzung mit der Flüssigkeit im wesentlichen luftundurchlässig wird.

Medizinische Flüssigkeitssysteme, wie z. B. Transfusionssysteme, sind normalerweise an denjenigen Stellen, an denen sich Öffnungen oder mögliche Austrittsstellen für die Flüssigkeit befinden, mit Schutzvorrichtungen bzw. Verschlusselementen versehen, um zu verhindern, dass die Flüssigkeitskanäle kontaminiert werden, bevor sie abdichtend miteinander gekuppelt werden. Derartige Flüssigkeitssysteme müssen jedoch, bevor sie mit Flüssigkeit gefüllt werden, entlüftet werden. Hierbei erweisen sich die Kontaminationsschutzeinrichtungen als hinderlich, weil sie nicht nur das Eindringen von Kontaminationen in die betreffende Leitung, sondern auch das Austreten von Luft aus dieser Leitung behindern. Die Entlüftung geschieht in der Regel dadurch, dass beim Einleiten von Flüssigkeit in das System auf der gegenüberliegenden Seite die vorher verschlossenen Auslaufkanäle geöffnet werden. Sobald an den Auslaufkanälen Flüssigkeit austritt, müssen die Auslaufkanäle unverzüglich wieder geschlossen werden. Bei dieser Manipulation sind Kontaminationen der Anschlussverbindung infolge der aus dem Auslaufkanal ausgetretenen Flüssigkeit praktisch nicht zu vermeiden. Die entweder einteiligen oder aus mehreren miteinander gekuppelten Elementen zusammengesetzten Flüssigkeitssysteme erfordern, soweit sie überhaupt zu entlüften sind, einen hohen Aufwand an Zeit und Überwachung für die Entlüftung, wobei stets die Gefahr besteht, dass unbeabsichtigt Flüssigkeit austritt und die betreffende Anschlussstelle kontaminiert wird.

Bekannt sind Entlüftungseinrichtungen mit einem Einsatz aus luftdurchlässigem porösem Material oder mit einer hydrophoben Schicht. Diese Entlüftungssysteme sind jedoch in beiden Richtungen luftdurchlässig und haben daher den Nachteil, dass nicht nur Luft aus dem Flüssigkeitssystem austreten, sondern auch nach dem Füllen des Flüssigkeitssystems Luft angesaugt werden kann, wenn z. B. in dem Flüssigkeitssystem ein Unterdruck auftritt.

Ferner ist aus der GB-A Nr. 2042918 eine Einrichtung der eingangs genannten Art bekannt. Hierbei bildet die hydrophile Schicht eine Filtermembran, die zwei Kammern eines Filtergehäuses voneinander trennt. Die hydrophobe Membran ist Bestandteil einer Entlüftungseinrichtung. Die beiden Membranen sind nicht in Kontakt miteinander.

Der Erfindung liegt die Aufgabe zugrunde, eine Entlüftungseinrichtung zu schaffen, aus der beim Füllen des Flüssigkeitssystems die Luft austritt, während nach dem Füllen ein Lufteintritt in das System verhindert wird, wobei gleichzeitig das Austreten von Flüssigkeit verhindert wird.

Zur Lösung dieser Aufgabe ist bei einer Entlüftungseinrichtung der eingangs genannten Art erfindungsgemäss vorgesehen, dass die hydrophile Schicht und die hydrophobe Schicht in direktem gegenseitigen Flächenkontakt eine Öffnung des Flüssigkeitssystems verschliessen.

Bei der erfindungsgemässen Entlüftungseinrichtung kann beim Füllen des Systems Luft durch die beiden hintereinander angeordneten Schichten hindurch austreten. Wenn die Flüssigkeit die hydrophile Schicht erreicht, tritt sie in diese ein und bildet eine Barriere für den Luftdurchtritt. Sobald die Flüssigkeit die hydrophile Schicht benetzt hat, ist das Flüssigkeitssystem in dem erforderlichen Masse abgedichtet. Die hydrophobe Schicht verhindert das Ausfliessen von Flüssigkeit. Die hydrophile Schicht und die hydrophobe Schicht verschliessen in direktem gegenseitigen Flächenkontakt eine Öffnung des Flüssigkeitssystems. Die hydrophobe Schicht verhindert einerseits das Austreten von Flüssigkeit aus der hydrophilen Schicht in die Umgebung und bildet andererseits auch eine Kontaminationsschicht für die hydrophile Schicht.

Die hydrophile Schicht besteht vorzugsweise aus einem Filter mit etwa 0,2 µm Maschenweite.

Die erfindungsgemässe Entlüftungseinrichtung kann an Abschlusselementen des Flüssigkeitssystems angebracht sein, aber auch an solchen Komponenten des Flüssigkeitssystems, die eine anderweitige Funktion ausüben, wie z. B. an Filtern, Ventilen, Punktionskanülen, Verlängerungsleitungen oder Tropfkammern zur Selbstfüllung.

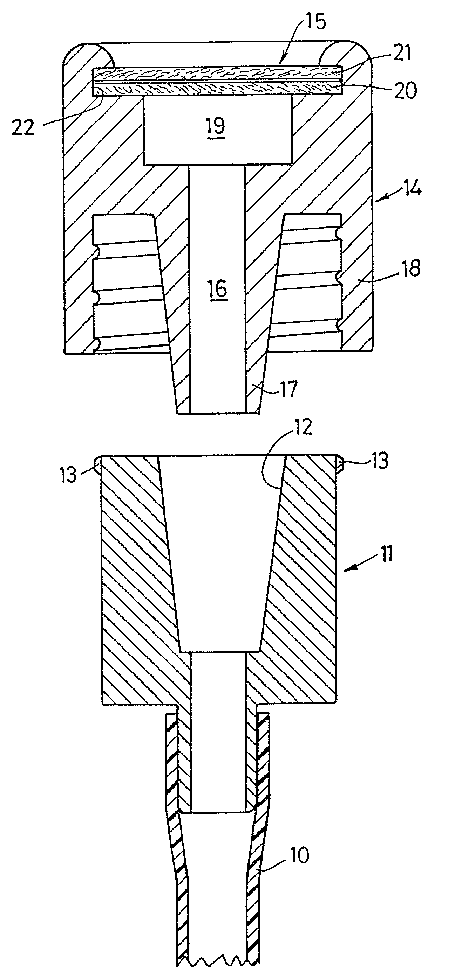

Im Folgenden wird unter Bezugnahme auf die einzige Figur die Zeichnung, in der ein schematischer Längsschnitt durch eine Entlüftungseinrichtung dargestellt ist, näher erläutert.

Eine Leitung 10 des Flüssigkeitssystems weist ein zylindrisches Anschlusselement 11 auf, das mit einer axialen konischen Bohrung 12 versehen ist. Von der Aussenseite des Anschlusselementes 11 stehen nach entgegengesetzten Richtungen Verriegelungselemente 13 ab. Das Anschlusselement 11, dessen rückwärtiger Stutzen abdichtend mit dem Schlauch 10 verbunden ist, dient zum Anschluss des Schlauches 10 an ein weiteres Anschlusselement 14, das am Ende eines Flüssigkeitskanals 16 die Entlüftungseinrichtung 15 aufweist. Der Flüssigkeitskanal 16 läuft axial durch einen Anschlusskonus 17 hindurch, welcher von einem mit inneren Gewindegängen versehenen Mantel 18 koaxial umgeben ist. Beim Verbinden der Anschlusselemente 11 und 14 wird der Anschlusskonus 17 in die ihm angepasste konische Bohrung 12 eingeführt, wobei die Verriegelungselemente 13 in die Gewindegänge des Mantels 18 eingedreht werden. Auf diese Weise wird der Kanal 16 unter Abdichtung gegen die Atmosphäre mit dem Inneren des Schlauches 10 verbunden.

Im rückwärtigen Bereich des Anschlusselementes 14 mündet der Kanal 16 in eine Kammer 19, welche von der Wand des Anschlusselementes 14 seitlich umgeben ist. Die Kammer 19 ist an ihrem rückwärtigen Ende durch eine hydrophile Schicht 20 begrenzt. Die Rückseite der hydrophilen Schicht 20 liegt an der hydrophoben Schicht 21 an. Die beiden Schichten 20 und 21, die flächenhaft gegeneinanderliegen, sind mit ihren Rändern in eine innere Ringnut 22 des Anschlusselementes 14 eingesetzt. Die Rückseite der hydrophoben Schicht 21 steht mit der Aussenluft in Verbindung.

Die Schichten 20 und 21 bestehen aus Fasern aus unterschiedlichen Materialien. Das Fasermaterial der Schicht 20 ist wasseransaugend, während dasjenige der Schicht 21 wasserabstossend ist. Wenn Flüssigkeit aus dem Schlauch 10 durch den Kanal 16 in die Kammer 19 gelangt, entweicht die von der Flüssigkeit verdrängte Luft durch die im trockenen Zustand durchlässige Schicht 20 und die ebenfalls luftdurchlässige Schicht 21 nach aussen. Erreicht die Flüssigkeit die hydrophile Schicht 20, dann werden die Fasern dieser Schicht 20 benetzt, so dass der Luftdurchgang durch die Schicht 20 blockiert oder mindestens stark erschwert wird. Die Schicht 21 wirkt flüssigkeitsabweisend und verhindert das Weiterdringen der Flüssigkeit aus der schliesslich gesättigten Schicht 20 nach aussen. Auf diese Weise bildet die Entlüftungseinrichtung 15 eine selbsttätig wirkende Sperre gegen das Austreten von Flüssigkeit und gegen das Eindringen von Luft.

## Patentansprüche

1. Entlüftungseinrichtung (15) für ein medizinisches Flüssigkeitssystem, mit einer das Flüssigkeitssystem abschliessenden luftdurchlässigen hydrophoben Schicht (21), und mit einer auf der der Flüssigkeit zugewandten Seite der hydrophoben Schicht (21) angeordneten Schicht (20) aus hydrophilem Material, die bei Benetzung mit der Flüssigkeit im wesentlichen luftundurchlässig wird, dadurch gekennzeichnet, dass die hydrophile Schicht (20) und die hydrophobe Schicht (21) in direktem gegenseitigen Flächenkontakt eine Öffnung des Flüssigkeitssystems verschliessen.

2. Entlüftungseinrichtung nach Anspruch 1, dadurch gekennzeichnet, dass die hydrophile Schicht (20) aus einem Filter mit etwa 0,2 μm Maschenweite besteht.

## Claims

1. Ventilation device (15) for a medical liquid system comprising an air-permeable hydrophobic layer (21) closing the liquid system, and a layer (21) of hydrophilic material provided at the hydrophobic layer (21) side confronted with the liquid and becoming substantially air-impermeable when wetted with the liquid, characterized in that the hydrophilic layer (20) and the hydrophobic layer (21) are closing in a direct mutual surface contact an opening of the liquid system.

2. Ventilation device according to Claim 1, characterized in that the hydrophilic layer (20) consists of a filter having a mesh width of about 0.2 μm.

## Revendications

1. Dispositif de ventilation (15) pour un système contenant un liquide médical, comportant une couche hydrophobe (21) perméable à l'air fermant le système à liquide et une couche (20) en matière hydrophile placée sur le côté de la couche hydrophobe (21) dirigé vers le liquide et devenant pratiquement imperméable à l'air quand elle est mouillée par le liquide, caractérisé en ce que la couche hydrophile (20) et la couche hydrophobe (21), en contact direct par leurs faces en regard, obturent un orifice du système à liquide.

2. Dispositif de ventilation selon la revendication 1, caractérisé en ce que la couche hydrophile (20) est constituée d'un filtre ayant une ouverture de maille d'environ 0,2 μm.